# EUROPÄISCHE PATENTSCHRIFT

(11) **EP 1 742 636 B1**
(45) Veröffentlichungstag und Bekanntmachung des Hinweises auf die Patenterteilung: **10.04.2013**
(21) Anmeldenummer: 05735958.0
(22) Anmeldetag: 05.05.2005
(51) Int. Cl.: A61K 31/498, A61K 31/575, A61P 35/00

(54) **Präparat zur Behandlung von Krebs**
Preparation for the treatment cancer
Preparation pour le traitement du cancer

(30) Priorität: 05.05.2004 CH 802042004
(43) Veröffentlichungstag der Anmeldung: 17.01.2007
(73) Patentinhaber: Immunopharm Aktiengesellschaft, 9496 Balzers (LI)
(72) Erfinder: GRIMM, Christine, 78262 Gailingen (DE); EHRENBERGER, Klaus, A-1090 Wien (AT); THURNHER, Dietmar, A-1160 Wien (AT)
(74) Vertreter: Schüssler, Andrea
(86) Internationale Anmeldenummer: PCT/CH2005/000254
(87) Internationale Veröffentlichungsnummer: WO 2005/105098

(56) Entgegenhaltungen:
- WO-A-99/50254
- DE-A- 3 507 721
- DE-A- 10 018 098
- US-A- 5 278 296
- US-B2- 6 573 265
- PATENT ABSTRACTS OF JAPAN Bd. 0071, Nr. 43 (C-172), 22. Juni 1983 (1983-06-22) & JP 58 057317 A (MITSUBISHI KASEI KOGYO KK), 5. April 1983 (1983-04-05)
- TSURUO T. ET AL: "Increased accumulation of vincristine and Adriamycin in drug-resistant P388 tumor cells following incubation with calcium antagonists and calmodulin inhibitors." CANCER RESEARCH, 42/11 (4730-4733). CODEN: CNREA8, 1982, XP008038938
- TSURUO T ET AL: "Potentiation of vincristine and adriamycin effects in human hemopoietic tumor cell lines by calcium antagonists and calmodulin inhibitors" CANCER RESEARCH, AMERICAN ASSOCIATION FOR CANCER RESEARCH, BALTIMORE, MD, US, Bd. 43, Nr. 5, Mai 1983 (1983-05), Seiten 2267-2272, XP002116183 ISSN: 0008-5472
- MAIER G ET AL: "ANTITUMOR ACTIVITY AND INDUCTION OF APOPTOSIS BY WATER-SOLUBLE DERIVATIVES OF 7BETA-HYDROXYCHOLESTEROL IN HUMAN COLON CARCINOMA CELL LINES" ANTICANCER RESEARCH, HELENIC ANTICANCER INSTITUTE, ATHENS,, GR, Bd. 19, Nr. 5B, 1999, Seiten 4251-4256, XP008039105 ISSN: 0250-7005
- CHAN, W. Y. ET AL: "Antiproliferative and teratogenic activities of the bishemisuccinates of 7.alpha.- hydroxycholesterol and 7.beta.- hydroxycholesterol" GENERAL PHARMACOLOGY , 25(4), 767-72 CODEN: GEPHDP; ISSN: 0306-3623, 1994, XP008039027

## Beschreibung

### Gebiet der Erfindung

Die vorliegende Erfindung betrifft ein Präparat zur Behandlung von Krebs gemäss Oberbegriff des Anspruchs 1.

### Stand der Technik

In der EP-A-594 581 ist erwähnt, dass das Delta-Cholesterol-3β,7β -diol erfolgreich in der Behandlung von Krebskrankheiten unterschiedlicher Phenotypen eingesetzt worden sei. Das Delta-Cholesterol-3β,7 β -diol wird in der Thymusdrüse gebildet und ist eine universelle Botensubstanz mit einer eigenen Immunabwehr. Das Delta-Cholesterol-3β,7β -diol verdankt seine Wirksamkeit, welche nur gegen bösartige Zelloberflächen gerichtet ist, der Tatsache, dass es unspezifisch an LDL-Cholesterin gebunden ist, welches für den Transport von Cholesterin in die Zellen verantwortlich ist. Da die Rezeptoren von LDL-Lipoproteinen auf der Oberfläche von Krebszellen degeneriert sind (Veränderung der räumlichen Struktur), bewirkt Delta-Cholesterol-3β,7β -diol eine Blockierung der degenerierten Rezeptoren. Es wurde gefunden, dass das Delta-Cholesterol-3β,7β -diol vollständig untoxisch ist und gesunde Zellen nicht angreift.

Die US 6,573,265 beschreibt die Verwendung von 1-Diethylaminoethyl-3-Chinoxalin-2-on Derivaten für die Behandlung von Schizophrenie, Lähmung der Gesichtsnerven, Redestörung infolge eines Schlaganfalls, Hörsturz, sowie von Dickdarm-und Hautkrebs. Die Wirkung der 1-Diethylaminoethyl-3-Chinoxalin-2-on Derivaten wird auf deren stark antioxidative Eigenschaften zurückgeführt. Behandelbar sind insbesondere Krankheiten, welche durch freie Radikale des Sauerstoffmetabolismus verursacht werden. Gemäss diesen Untersuchungen sind die 1-Diethylaminoethyl-3-Chinoxalin-2-on Derivate effiziente Radikalfänger für Hydroxyl-, Peroxyl- und Peroxynitrit-Radikale, welche für eine Mehrzahl von Krankheiten verantwortlich sind. Beschrieben ist in der US 6,573,265 auch die Verabreichung von Caroverin (1-Diethylaminoethyl-3-Chinoxalin-2-on-Derivaten) in Verbindung mit einer herkömmlichen Chemotherapie unter Verwendung der Präparate Irinothecam (anti-neoplastisches Mittel) und Oxaliplatin zur Behandlung von Dickdarm-, Hirn- und Rachentumoren. Die Wirkung von Caroverin bei bestimmten Tumoren wird damit erklärt, dass durch die hohe antioxidative Wirkung von Caroverin freie Radikale des Sauerstoff-Zellmetabolismus eliminiert werden und damit eine fortdauernde DNA - Schädigung, welche Krebs verursacht, verhindert wird, d.h. es wurde davon ausgegangen, dass Caroverin keine direkte Wirkung auf Krebszellen hat.

Es ist auch bekannt, dass 1-Diethylaminoethyl-3-Chinoxalin-2-on Derivate aktive Verbindungen sind, welche einen Einfluss auf den Zu- und Abfluss von Antikrebsmitteln in und aus Zellen kontrollieren (Derwent Abstract, Publikation No. AN 83-45789 K). Durch die Zugabe der 1-Diethylaminoethyl-3-Chinoxalin-2-on Derivate kann eine hohe Konzentration der Krebsmittel in den Zellen für eine längere Zeit erhalten bleiben. Es ist jedoch bemerkenswert, dass gemäss obiger zitierter Publikation den 1-Diethylaminoethyl-3-Chinoxalin-2-on Derivaten selbst keine eigentliche krebshemmende Wirkung zugeschrieben wird.

Tsuro et al. (Cancer Research, 42/11 (4730 - 4733 und 43, 2267 - 2272) haben festgestellt, dass einige Calcium-Antagonisten und Calmodulin-Inhibitoren die intrazelluläre Konzentration von Vincristine und Adriamycin erhöhen können, indem der Abfluss der Wirksubstanzen aus den Zellen verhindert wird. Solche Calcium-Antagonisten und Calmodulin-Inhibitoren wirken dabei als sogenannte Modulatoren. Gemäss ihren Untersuchungen sind die Calcium-Antagonisten nicht direkt zytotoxisch für die Tumorzellen. Dies wird auch von anderer Seite bestätigt (s. Menke et al. in Dtsch. med. Wschr. 113 (1986), 1728-1732). Ausserdem scheint die Effektivität der Calcium-Antagonisten zur Überwindung der Vincrystine- oder Adriamycin- Resistenz von Tumorzellen nicht direkt mit deren Effektivität als Calmodulin-Inhibitoren verbunden zu sein.

WO 99/50254 betrifft die Verbindungsklasse der Chinoxalone ganz allgemein und deren mögliche Verwendung zur Behandlung verschiedener Krankheiten. Unter anderem ist erwähnt, dass die Verbindungsklasse sich zur Behandlung von Krebs eigne.

Gerhard Maier et al. (in Anticancer Research, 19, 4251-4256 (1999)) untersuchten die Zytotoxizität von 7β-Hydroxycholesterol und von zwei wasserlöslichen Derivaten von 7β-Hydroxycholesterol auf verschiedene Darmkrebszelllinien. Durch die Untersuchungen konnte nachgewiesen werden, dass die 7β-Hydroxycholesterole zum Zelltod der Krebszellen führten.

Es ist im Weiteren bekannt, dass die unkrontrollierte Proliferation von Tumorzellen unterschiedliche Ursachen haben kann und dass es verschiedene Mechanismen gibt, nach welchen Krebszellen den "programmierten" Zelltod von Körperzellen verhindern. Herkömmliche in der Chemotherapie eingesetzte Krebsmittel zielen darauf ab, die Krebszellen abzutöten. Wegen der meist begrenzten Selektivität der eingesetzten Chemikalien und der Unmöglichkeit, die Verbindung nur lokal wirken zu lassen, werden dabei regelmässig auch gesunde Körperzellen in Mitleidenschaft gezogen. Dies bedeutet, dass solche Chemotherapien mit entsprechenden starken negativen Nebeneffekten einhergehen.

Tsuro et al. offenbaren, dass sich in Arzneimittel-resistenten P388-Tumorzellen in Folge einer Inkubation mit Calcium-Antagonisten und Calmodulin-Inhibitoren Vincristin und Adriamycin vermehrt anhäufen (Tsuro et al., 1982, Cancer Research, 42/11:4730-4733).

Weiterhin zeigen Tsuro et al. die Wirkungen der Potenzierung von Vincristin und Adriamycin in menschlichen hämatopoetischen Tumor-Zelllinien durch Calcium-Antagonisten und Calmodulin-Inhibitoren (Tsuro et al., Mai 1983, Cancer Research, American Association for Cancer Research, Baltimore, MD, US, Bd. 43, Nr. 5, S. 2267-2272).

Neben der Verhinderung des "programmierten" Zelltods ist für das Wachstum eines Tumors auch erforderlich, dass die Tumorzellen mit ausreichend Blut versorgt werden. In dieser Hinsicht spielt der vaskuläre endotheliale Wachstumsfaktor (vascular endothelial growth factor (VEGF)) eine entscheidende Rolle. Der VEGF induziert eine Neovaskularisation, was die Versorgung von wachsenden Tumoren mit Blut erlaubt.

### Aufgabe der Erfindung

Es ist deshalb Aufgabe der vorliegenden Erfindung, verbesserte Krebsmittel bereitzustellen. Insbesondere ist es ein Ziel, ein Krebsmittel bereitzustellen, welches Krebszellen an unterschiedlichen Stellen resp. mittels unterschiedlicher Wirkmechanismen angreift. Noch ein Ziel ist es, ein Präparat zur Behandlung von Krebs mit weniger Nebenwirkungen bereitzustellen.

### Beschreibung

Ein Präparat zur Behandlung von Krebs, kann als aktive Substanzen mindestens ein 1-Diethylaminoethyl-3-Chinoxalin-2-on Derivat der Formel 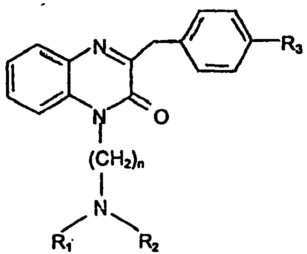 worin R1 und R2 unabhängig voneinander Wasserstoff, Methyl-, Ethyl, Propyl-, Butyl, oder R1 und R2 zusammen eine Cycloalkylverbindung sind;
R3 ist Methoxy, Ethoxy, Hydroxy, Wasserstoff, C1-C4 Alkyl Halogen; und
n=1,2 oder 3,
oder eines pharmazeutisch verträglichen Salzes der erwähnten Derivate,
sowie
eine wirksame Menge eines zytotoxischen Krebsmittels mit der Ausnahme von Cisplatin, Irinotecan, Vincristine und/oder Adriamycin. Es wurde heraus gerunden, dass 1-Diethylaminoethyl-3-Chinoxalin-2-on Derivate imstande sind, die Proliferation der Tumorzellen einzuschränken oder ganz zu verhindern. Dadurch ist es möglich, das Wachstum des Tumors zu stoppen. Im Unterschied zu den bisherigen Erkenntnissen haben -Diethylaminoethyl-3-Chinoxalin-2-on Derivate doch eine direkt krebshemmende Wirkung. Deren Wirkung beruht darauf, dass die Blutzufuhr zu den Krebszellen unterbunden werden kann. In Verbindung mit zytotoxischen resp. zytostatischen Krebsmitteln mit apoptotischer Wirkung ergibt sich überraschenderweise ein nicht vorhersehbarer synergistischer Effekt. Durch die Kombination von - Diethylaminoethyl-3-Chinoxalin-2-on Derivaten und zytotoxischen Krebsmitteln mit direkt apoptotischer Wirkung wurde somit ein effizientes und gut verträgliches Kombinationspräparatgeschaffen, welches zu einer Rückbildung der Tumore führt. Das Kombinationspräparat kann gegen verschiedenste Tumore eingesetzt werden. Das Präparat ist auch gegen krebsartige Plattenepithelzellen wirksam, deren Bekämpfung als sehr schwierig gilt. Die bisherigen Studien haben gezeigt, dass überraschenderweise die Konzentration des zytostatischen Krebsmittels verringert werden kann, wenn es zusammen mit Diethylaminoethyl-3-Chinoxalin-2-on Derivaten, vorzugsweise Caroverin, eingesetzt wird. Est ist denkbar, zwei oder mehrere zytotoxische resp. zytostatische Krebsmitteln in Verbindung mit -Diethylaminoethyl-3-Chinoxalin-2-on Derivaten, vorzugsweise Caroverin oder Caroverin-Derivaten, einzusetzen. Als zytotoxische Krebsmittel in Kombination mit Caroverin-Derivaten werden insbesondere Verbindungen aus der Klasse der Oxysterole oder der Polyphenole, oder eine Mischung enthaltend Selen und L-Cystein und/oder L-Glutathion, oder eine Mischung enthaltend Vitamine des B-Komplexes, insbesondere mit den Vitaminen Bi, und/oder B₆ und/oder B₁₂ vorgeschlagen.

Erfindungssemäße Präparat enthält als zytostatisches Krebsmittel mindestens eine Verbindung aus der Klasse der Oxysterole. Oxysterole haben eine zytostatische Wirkung, und es konnte in Kombination mit den 1-Diethylaminoethyl-3-Chinoxalin-2-on Derivaten überraschenderweise ein ausgeprägter synergestischer Effekt festgestellt werden. Erfindugsgemäß wird als Oxysterol 7β-Hydroxycholesterol oder ein pharmazeutisch verträgliches Salz davon eingesetzt (z.B. 7β-Hydroxycholesterol -bis-hemisuccinate-DiNatrium-Salz oder 7β-Hydroxycholesterol -bis-hemisuccinate-diethanolaminoate). Dabei können eine oder beide Hydroxylgruppen mit unterschiedlichen Substituenten, insbesondere mit Mono-, Bi- oder Tricarbonsäuren, substitiuiert sein. Durch geeignete Substituierung des 7β-Hydroxycholesterol kann dessen Wasserlöslichkeit, Resorptionsvermögen etc. beeinflusst werden. Entsprechend kann das eingesetzte Oxysterol eine Verbindung nachfolgender Strukturformeln sein:

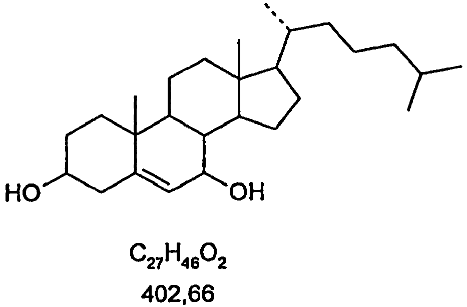

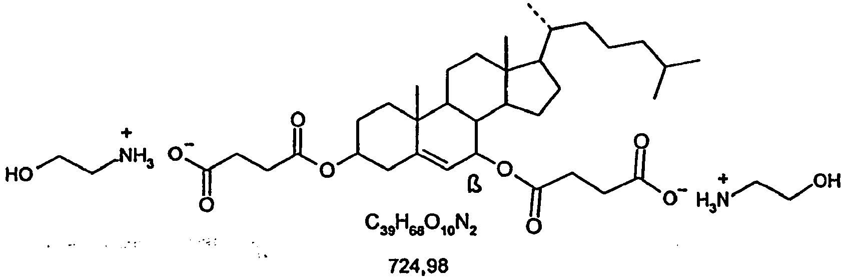

Alternativ wird beschrieben, dass als Zytostatikum eine Verbindung aus der Klasse der Polyphenole, wie Resveratrol, Curcumin, Quercetin, Gingerolen oder oligomere Proanthocyanidine (bekannt unter der Abkürzung: OPC) eingresetz werden kann. Ferner wird beschrieben, dass als Zytostatikum eine wirksame Menge.einer Mischung aus Selen und L-Cystein und/oder Glutathion einzusetzen Dabei können geringe Menge Selen (1 bis 50 µg) mit L-Cystein resp. Glutathion (10 bis 100 mg) kombiniert sein. Alternativ wird beschrieben, als Krebsmittel eine wirksame Menge des Vitamin B-Komplexes, enthaltend insbesondere die Vitamine B₁, und/oder B₆ und/oder B₁₂ in Verbindung mit Caroverin einzusetzen.

Als 1-Diethylaminoethyl-3-Chinoxalin-2-on Derivat wird erfindugsgemäß Verbindung der Formel 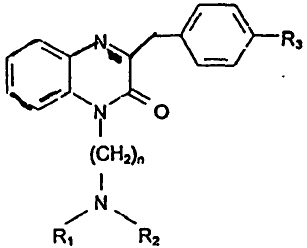 eingesetzt, worin R1 und R2 eine Ethyl-Gruppe; n=2 und R3 eine Methoxy-Gruppe sind, sodass das Molekül 1-Diethylaminoethyl-3-(p-methoxybenzyl)-1,2-dihydro-Chinoxalin-2-on (INN: Caroverin) oder ein pharmazeutisch verträgliches Salz ist.

Gegenstand der vorliegenden Erfindung ist somit die Verwendung des erfindungsgemäßen 1-Diethylaminoethyl-3-Chinoxalin-2-on Derivats der Formel 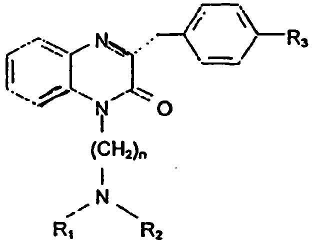 worin R1 und R2 eine Ethyl, -Gruppe ist;
R3 ist Methoxy; und
n=2
oder eines pharmazeutisch verträglichen Salzes davon,
sowie
einer wirksamen Menge des erfindungsgemäßen zytostatischen Krebsmittels zur Herstellung eines Mittels zur Behandlung von Krebs, insbesondere von Dickdarmkrebs, Schleimhautkarzinomen, Dickdarm- oder Brustkrebs.

Die Erfindung wird nachfolgend unter Bezugnahme auf die Figuren beispielhaft beschrieben. Es zeigt:
- Fig. 1: Dosisabhängige Ansprechkurve von 7β-Hydroxycholesterol als Funktion der verabreichten Menge 7β-Hydroxycholesterol nach einer Behandlungsdauer von 72 Stunden;
- Fig. 2: Eine graphische Darstellung des synergistischen Effekts von 7β-Hydroxycholesterol in Kombination mit Cisplatin;
- Fig. 3: Dosisabhängige Ansprechkurve von Caroverin gegen die Krebszelllinie SCC 25;
- Fig. 4: Ansprechkurve von Caroverin (quantitativ: Ermittlung des EC50-Wertes)
- Fig. 5: Eine graphische Darstellung der Wirksamkeit von Caroverin in Kombination mit Cisplatin;
- Fig. 6: Eine graphische Darstellung des synergistischen Effekts von 7β-Hydroxycholesterol und Caroverin bezüglich der Krebslinie SCC25 als Funktion der Konzentration;
- Fig. 7: Wirksamkeit von 10 µM Caroverin und 10 µM Hydroxycholesterol allein und in Kombination;
- Fig. 8: Wirksamkeit von 20 µM Caroverin und 10 µM Hydroxycholesterol allein und in Kombination;
- Fig. 9: Eine graphische Darstellung des synergistischen Effekts von 7β-Hydroxycholesterol und Caroverin bezüglich der Krebszelllinie SCC9 als Funktion der Konzentration;
- Fig. 10: Wirksamkeit von 10 µM Caroverin und 10 µM Hydrocholesterol allein und in Kombination bezüglich der Krebszelllinie SCC9.

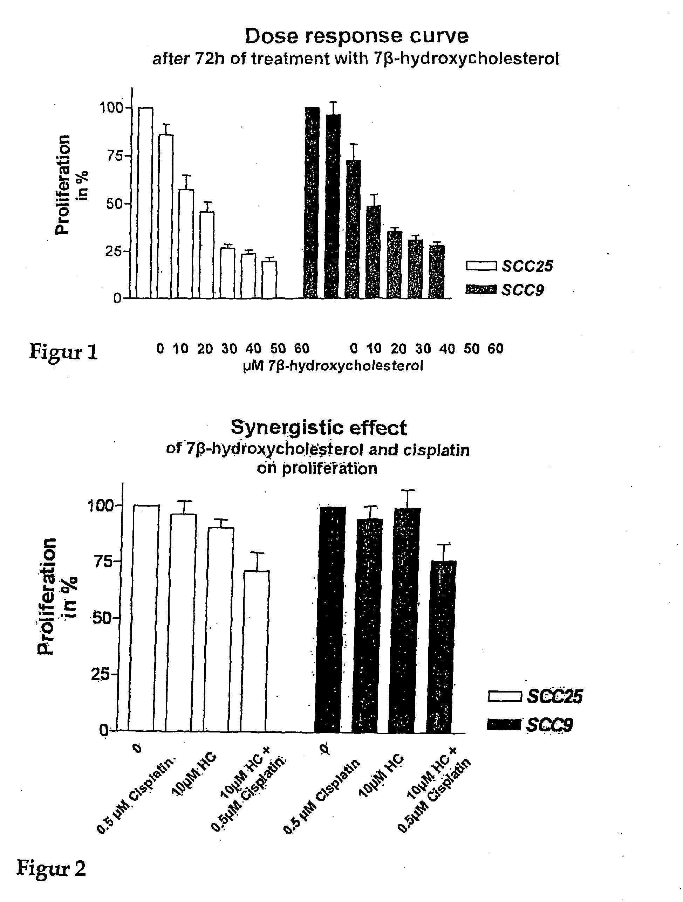

Untersucht wurde die biologische Aktivität von 7β-Hydroxycholesterol und Caroverin allein, als auch in Verbindung miteinander. Die Wirksamkeit wurde anhand der Kopf- und Halstumorzelllinien SCC 9 und SCC 25 überprüft. Figur 1 zeigt, dass 7β-Hydroxycholesterol (HC) gegen beide Zelllinien SCC 9 und SCC 25 wirksam ist. Dabei nimmt die Wirksamkeit von 7β-Hydroxycholesterol mit Zunahme der verabreichten Menge deutlich zu.

Der Figur 2 kann entnommen werden, dass 7β-Hydroxycholesterol in geringen Dosen einen synergistischen Effekt mit Cisplatin hat.

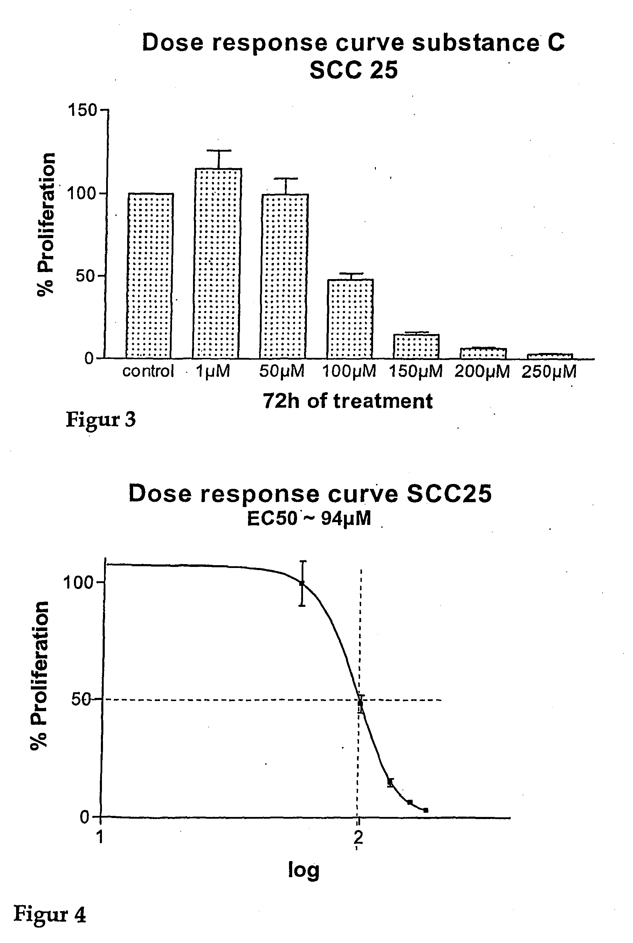

Aus der Figur 4 ist diejenige Konzentration ersichtlich, bei welcher die Proliferation um 50 % gehemmt ist.

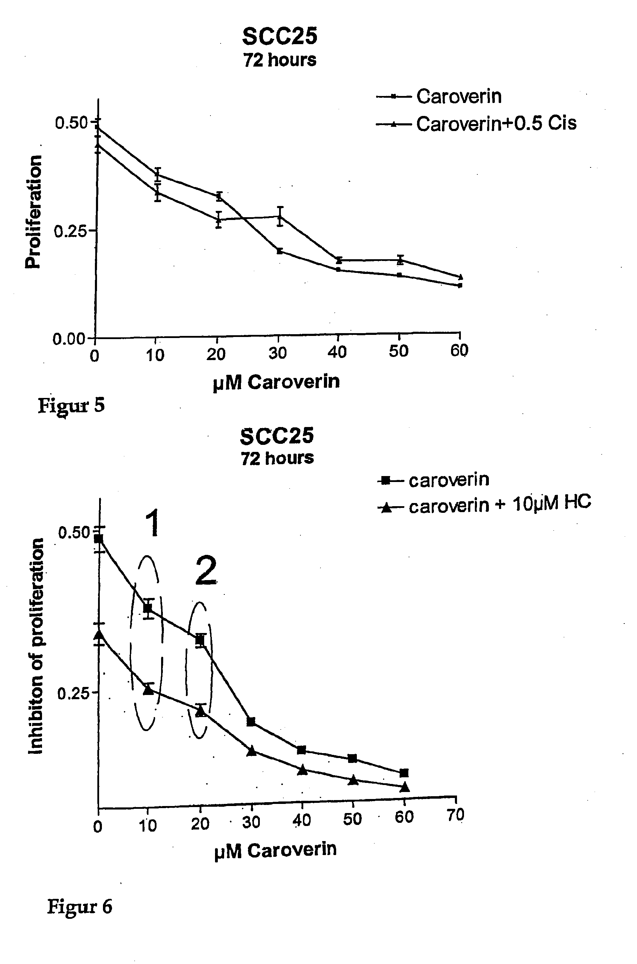

Eine synergistische Wirkung von Caroverin in Verbindung mit Cisplatin konnte jedoch nicht nachgewiesen werden (Fig. 5).

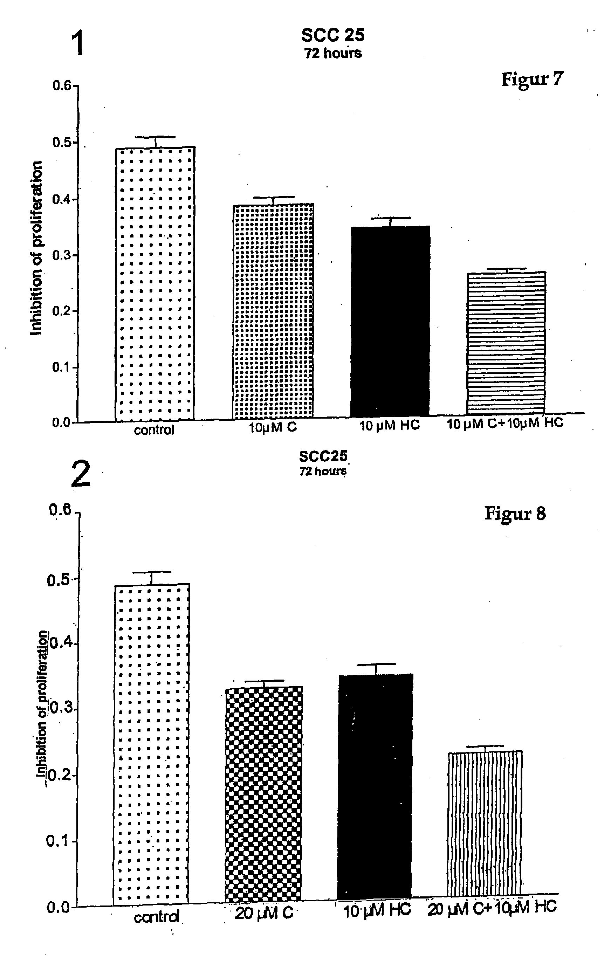

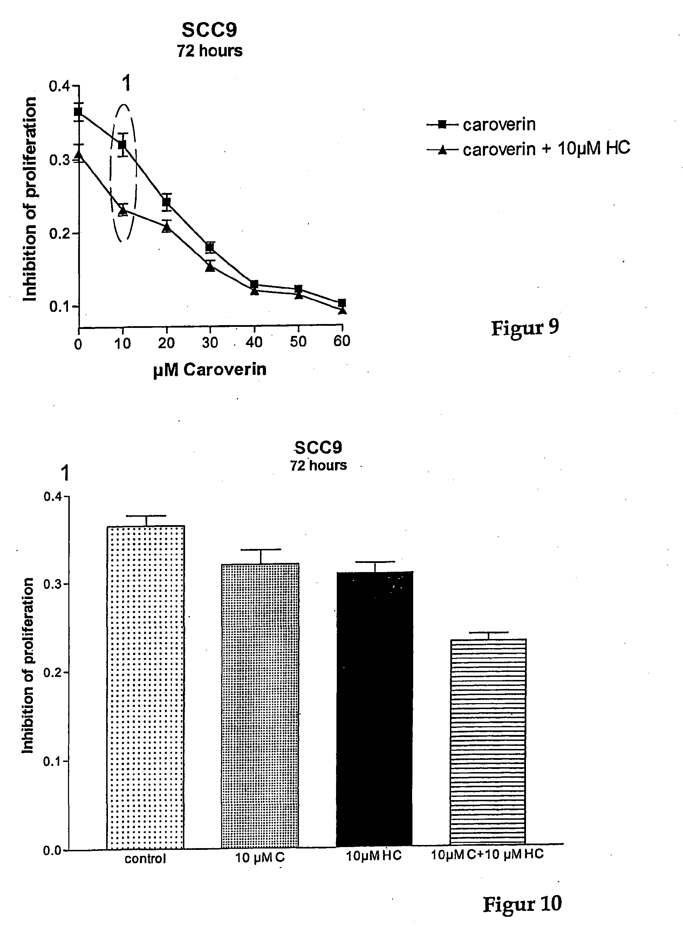

Zur Überraschung der Erfinder konnte jedoch in Experimenten nachgewiesen werden, dass ein Kombinationspräparat enthaltend 7β-Hydroxycholesterol und Caroverin sowohl gegen die Krebzelllinien SCC9 als auch SCC25 wirksam ist (Figuren 6 bis 10). Die Wirksamkeit des Kombinationspräparats ist konzentrationsabhängig. Die besten Resultate werden bei einer Konzentration bis ca. 30 µM Caroverin erzielt.

Das erfindungsgemässe Kombinationspräparat kann eingesetzt werden zur Behandlung von krebsartigen Plattenepithelzellen, welche beispielsweise im Kopf- und Halsbereich vorkommen. Dies ist überraschend, da Plattenepithelzellen bekanntermassen die am schwierigsten zu bekämpfenden Krebszellen sind. Folglich kann davon ausgegangen werden, dass das Präparat auch bei anderen, weniger hartnäckigen Krebsarten eingesetzt werden kann. Insbesondere können mit dem Präparat Schleimhautkarzinome, Dickdarm-und Brustkrebs effizient behandelt werden.

Das Präparat kann intravenös, topisch, oral, parenteral oder transdermal verabreicht werden. Vorzugsweise wird Caroverin in Mengen von 20 bis 200 mg pro Tag, vorzugsweise zwischen 60 und 160 mg pro Tag verabreicht. Hydroxycholesterol wird vorzugsweise in Mengen von 5 bis 50 mg pro Tag, vorzugsweise 15 bis 30 mg pro Tag verabreicht. Vorzugsweise wird 7β-Hydroxycholesterol in neutraler Form (nicht als Salz) eingesetzt. Es hat sich nämlich in klinischen Studien gezeigt, dass die Wirksamkeit des Kombinationspräparats deutlich besser ist, wenn 7β-Hydroxycholesterol als neutrales Molekül eingesetzt wird.

Die vorliegende Erfindung betrifft ein gegen Krebszellen wirkendes Kombinationspräparat enthaltend als aktive Substanzen mindestens ein 1-Diethylaminoethyl-3-(p-methoxybenzyl)-1,2-dihydro-chinoxalin-2-on (INN:Caroverin) 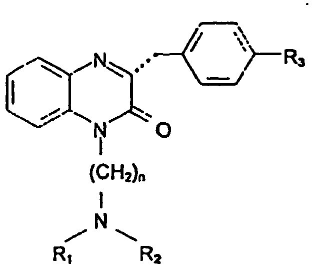 worin R1 und R2 eine Ethyl, -Gruppe ist;
R3 ist Methoxy, und n=2
oder eines pharmazeutisch verträglichen Salzes davon,
sowie
eine wirksame Menge des zytostatischen Krebsmittels, 7β-Hydroxycholesterol oder eines pharmazeutisch verträglichen Salzes davon.

## Patentansprüche

1. Präparat zur Behandlung von Krebs enthaltend als aktive Substanzen
1-Diethylaminoethyl-3-(p-methoxybenzyl)-1,2-dihydro-Chinoxalin-2-on (INN: Caroverin) oder ein pharmazeutisch verträgliches Salz davon,
sowie
7β-Hydroxychofesterol oder ein pharmazeutisch verträgliches Salz davon.

2. Präparat zur Anwendung nach Anspruch 1, **dadurch gekennzeichnet, dass** das eingesetzte 7β-Hydroxycholesterol eine Verbindung folgender Strukturformel oder ein pharmazeutisch verträgliches Salz davon ist:

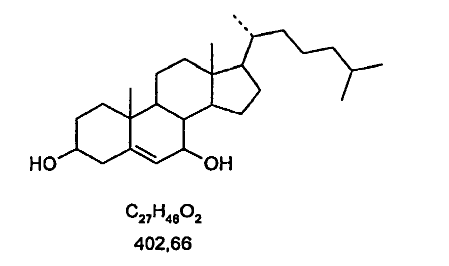

3. Präparat zur Anwendung nach Anspruch 1, **dadurch gekennzeichnet, dass** das eingesetzte 7β-Hydroxycholesterol 7β-Hydroxycholesteryl-bishemisuccinat-di-ethanolamin Salz mit der Formel 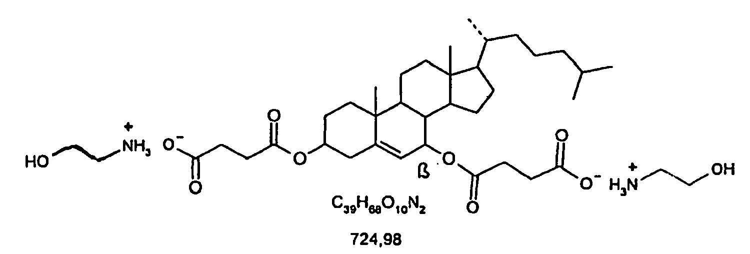 ist.

4. Präparat zur Anwendung gemäß einem der Ansprüche 1-3, **dadurch gekennzeichnet, dass** der zu behandelnde Krebs Brustkrebs ist.

5. Präparat zur Anwendung gemäß einem der Ansprüche 1-3, **dadurch gekennzeichnet, dass** der zu behandelnde Krebs Dickdarmkrebs ist.

6. Präparat zur Anwendung gemäß einem der Ansprüche 1-3, **dadurch gekennzeichnet, dass** der zu behandelnde Krebs ein Schleimhautkarzinom ist.

## Claims

1. Compound for the treatment of cancer comprising as active substances
1-diethylaminoethyl-3-(p-methoxybenzyl)-1,2-dihydro-quinoxaline-2-one (INN: Caroverine) or a pharmaceutically acceptable salt thereof,
and
7 β-hydroxycholesterol or a pharmaceutically acceptable salt thereof.

2. Compound for the use according to claim 1, **characterized in that** the 7 β-hydroxycholesterol utilized is a compound of the following structural formula or a pharmaceutically acceptable salt thereof

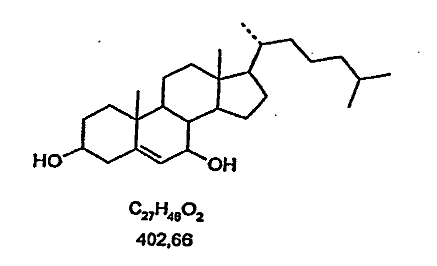

3. Compound for the use according to claim 1, **characterized in that** the 7 β-hydroxycholesterol utilized is a 7 β-hydroxycholesteryl-bishemisuccinat-diethanolamine salt of the formula

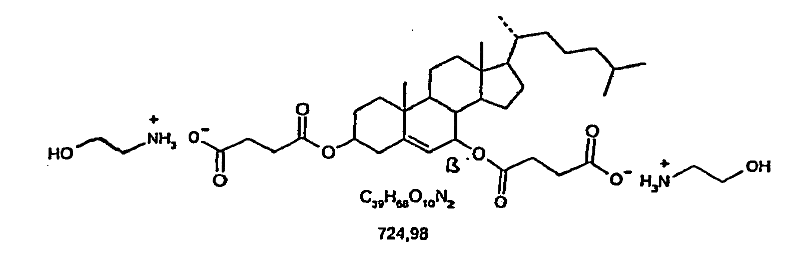

4. Compound for the use according to any one of the claims 1-3, **characterized in that** the cancer to be treated, is a breast cancer.

5. Compound for the use according to any one of the claims 1-3, **characterized in that** the cancer to be treated, is a colon cancer.

6. Compound for the use according to any one of the claims 1-3, **characterized in that** the cancer to be treated, is a mucosal carcinoma.

## Revendications

1. Préparation pour le traitement de cancer contenant comme substances actives
1-diéthylaminoéthyl-3-(p-méthoxybenzyl)-1,2-dihydro-chinoxalin-2-on (INN: carovérine) ou un sel acceptable pharmaceutiquement de ce dernier,
ainsi que
7β-hydroxycholestérol ou un sel acceptable pharmaceutiquement de ce dernier.

2. Préparation pour l'application selon la revendication 1, **caractérisée par le fait que** le 7β-hydroxycholestérol utilisé est une liaison de la formule de structure suivante ou un sel acceptable pharmaceutiquement de cette dernière:

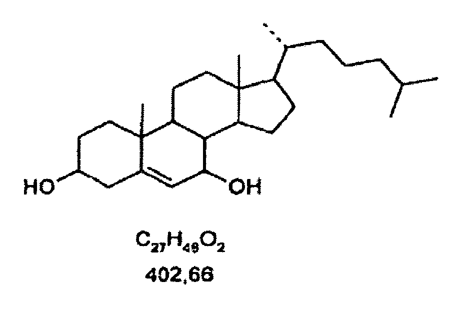

3. Préparation pour application selon la revendication 1, **caractérisée par le fait que** le 7β-hydroxycholestérol est un sel de 7β-hydroxycholestéryl-bishémisuccinat-di-éthanolamine à la formule

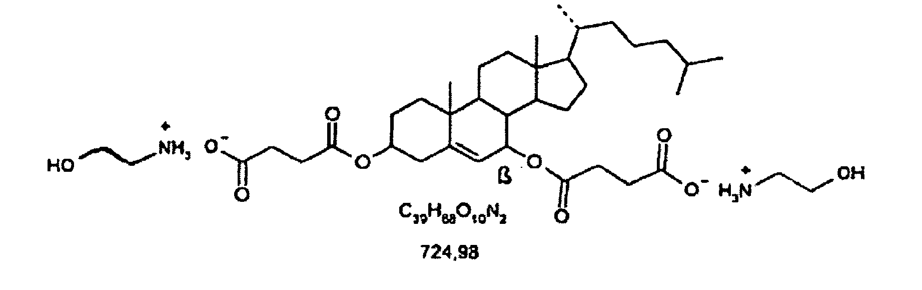

4. Préparation pour application selon l'une des revendications 1 à 3, **caractérisée par le fait que** le cancer à traiter est le cancer du sein.

5. Préparation pour application selon l'une des revendications 1 à 3, **caractérisée par le fait que** le cancer à traiter est le cancer du gros intestin.

6. Préparation pour application selon l'une des revendications 1 à 3, **caractérisé par le fait que** le cancer à traiter est un carcinome de la muqueuse.
